# EUROPEAN PATENT SPECIFICATION

(11) **EP 4 460 309 B1**
(45) Date of publication and mention of the grant of the patent: **19.08.2026**
(21) Application number: 23702218.1
(22) Date of filing: 05.01.2023
(51) Int. Cl.: A61K 31/502, A61K 31/573, A61K 31/58, A61K 31/53, A61K 45/06, A61P 21/00

(54) **USE OF 5-AMINO-2,3-DIHYDRO-1,4-PHTHALAZINEDIONE IN THE TREATMENT OF CONGENITAL MUSCULAR DYSTROPHIES**
VERWENDUNG VON 5-AMINO-2,3-DIHYDRO-1,4-PHTALAZINDION ZUR BEHANDLUNG VON KONGENITALEN MUSKELDYSTROPHIEN
UTILISATION DE 5-AMINO-2,3-DIHYDRO-1,4-PHTALAZINEDIONE DANS LE TRAITEMENT DES DYSTROPHIES MUSCULAIRES CONGÉNITALES

(30) Priority: 07.01.2022 EP 22000008
(43) Date of publication of application: 13.11.2024
(73) Proprietor: MetrioPharm AG, 8021 Zürich (CH)
(72) Inventor: BRYSCH, Wolfgang, 13505 Berlin (DE); BREMBECK, Felix, 10785 Berlin (DE); SCHUMANN, Sara, 14554 Seddiner See (DE); VON WEGERER, Jörg, 13597 Berlin (DE)
(74) Representative: Gruber, Daniel
(86) International application number: PCT/EP2023/000002
(87) International publication number: WO 2023/131579

(56) References cited:
- WO-A1-2017/202496
- WO-A1-2021/249667
- WO-A1-2022/008093
- JP-A- 2017 537 958
- US-A1- 2009 018 137
- US-A1- 2020 369 624
- US-A1- 2021 315 873
- US-B1- 6 953 799
- MESA L E ET AL: "Steroids in duchenne muscular dystrophy - Deflazacort trial", NEUROMUSCULAR DISORDERS, ELSEVIER LTD, GB, vol. 1, no. 4, 1 January 1991 (1991-01-01), pages 261 - 266, XP024402868, ISSN: 0960-8966, [retrieved on 19910101], DOI: 10.1016/0960-8966(91)90099-E

## Description

### FIELD OF THE INVENTION

The present invention relates to 5-amino-2,3-dihydro-1,4-phthalazinedione or one of its pharmaceutically acceptable salts for use in the treatment of congenital muscular dystrophies, especially Duchenne muscular dystrophy or Becker muscular dystrophy. The invention relates in particular to 5-amino-2,3-dihydro-1,4-phthalazinedione sodium salt for use for said purposes. Also a pharmaceutical combination of 5-amino-2,3-dihydro-1,4-phthalazinedione or one of its pharmaceutically acceptable salts and a glucocorticoid for use in these indications is discloses although not claimed. Pharmaceutical compositions, advantageous formulation techniques and a method of treatment are disclosed.

### BACKGROUND OF THE INVENTION

Congenital muscular dystrophies (CMD) encompass a wide group of muscular disorders that are characterized by a progressive skeletal muscle weakness. They display histological muscle alterations and an abnormal muscle architecture. The severity and the course of the disease is variable. Within one year of age an early onset of muscular weakness starts. Until now more than 50 forms of CMD have been described. Most of them are due to a mutation in a gene encoding for a structural protein of skeletal muscle cells (cf. Falsaperla et al. (2016) It J Pediat 42: 78). These disorders are not very common. The most prominent among them and also the most feared of is Duchenne muscular dystrophy (DMD). DMD is an X chromosome-linked recessive progressive wasting disorder caused by a loss of function mutation in the dystrophin gene. Therefore, DMD is nearly exclusively limited to boys. DMD affects approximately 1 in 5,000 male births. Progressive muscle degeneration leads to loss of ambulation at 8 - 12 years of age. Premature death occurs normally between 20 - 30 years due to respiratory and cardiac failure (cf. Guiraud and Davies (2017) Current Opinion in Pharmacology 34: 36-48). Sometimes the life span can be extended with pharmacological treatment into their thirties. Treatment options are still very limited. The most promising approach was a gene therapy in which an intact dystrophin gene should be introduced into the genome. However, it showed only very moderate success, probably due to autoimmune reactions. Another approach is exon-skipping. Herein antisense oligonucleotides are administered that should skip defect parts of the dystrophin gene thus affording the generation of a version of dystrophin that is still truncated but halfway functional (cf. Dunckley et al. (1998) Human Mol Gen 7: 1083-1090). A moderate clinical improvement could be seen in studies with ataluren, while eteplirsen is still under clinical investigation. This method, however, requires periodic redelivery into the muscles. A systemic delivery route is still under investigation. Therefore, the standard approach is still a pharmacologic symptomatic treatment, trying to slow down the muscular deterioration thus extending the patient's life span as long as possible. The most common prescribed drugs for DMD include glucocorticoids, above all prednisone, prednisolone and deflazacort, as well as β₂ agonists such as salbutamol, anticonvulsants to control seizures and immunosuppressants.

Further approaches still under investigation are Na⁺/Ca²⁺ exchange pump inhibitors, NF-κB inhibitors, antioxidants, mitochondrial dysfunction modulators, HDAC (histone deacetylase) inhibitors, anti-fibrotic agents, myostatin inhibitors, TGF-β pathway anti-fibrotic agents and PDE5 inhibitors against muscle ischemia (cf. Guiraud and Davies (2017) Current Opinion in Pharmacology 34: 36-48).

Until now, glucocorticoids are still the relatively best treatment. Mesa et al: "Steroids in Duchenne muscular dystrophy - Deflazacort trial", NEUROMUSCULAR DISORDERS, ELSEVIER LTD, GB, vol. 1, no. 4, 1 January 1991, pages 261-266 discloses the ameliorating effect of Deflazacort against Duchenne Muscular Dystrophy. However, they entail a huge number of adverse side effects that harshly limit their long-time use. They comprise Cushing's syndrome, hypertonia, weight gain and obesity (particularly truncal obesity, including the so called "buffalo hump"), edema, puffiness of the face (moon face), potassium loss, muscle weakness, headache, thinning of the skin, easy bruising and slow wound healing, glaucoma, cataracts, stomach and duodenum ulcers, steroid-induced diabetes, loss of control in existing diabetes, osteoporosis (resulting in bone fractures), adrenal joint necrosis (in particular of the hip or the knee joint), psychiatric disturbances (e.g. depression, euphoria, insomnia, mood swings, personality changes), psychotic behavior, growth retardation in children, convulsions, an increased rate of infections, exacerbation of opportunistic infections (such as tuberculosis, *Herpes zoster* and *Pneumocystis* pneumonia), reduced effectiveness of antibiotics and vaccines.

*Mutatis mutandis* this holds true for all congenital muscular dystrophies.

Thus, there is a huge medical need to find a new medication that mitigates the symptoms of CMD, particularly of Duchenne muscular dystrophy or Becker muscular dystrophy, and that improves the life quality of the patients. Ideally, it should have the same therapeutic potency as glucocorticoids in these diseases but without these strong adverse side effects in order to replace glucocorticoid treatment or to allow for a considerable reduction of glucocorticoids when combined.

Surprisingly, this task is solved by 5-amino-2,3-dihydro-1,4-phthalazinedione or its pharmaceutically acceptable salts.

### DESCRIPTION OF THE INVENTION

The present application is defined in the appended claims.

Any references in the description to methods of treatment refer to the compounds, pharmaceutical compositions and medicaments of the present invention for use in a method for treatment of the human (or animal) body by therapy (or for diagnosis).

5-amino-2,3-dihydro-1,4-phthalazinedione sodium salt is tested in a *mdx* mouse model.

These mice carry a nonsense mutation in the dystrophin gene, thus emulating a similar mutation in human Duchenne muscular dystrophy (and also Becker muscular dystrophy), as can be seen in Examples 1 and 2. It is assumed that the results in this model are predictive not only for DMD but for other congenital muscular dystrophies too.

The administration of 5-amino-2,3-dihydro-1,4-phthalazinedione sodium salt as a single drug leads to a significant improvement in physiologically relevant parameters such as muscle contractile properties and increased body weight, in comparison to control.

The comparison with therapeutic concentrations of the two widely used glucocorticoids in the treatment of DMD, prednisolone and deflazacort, showed that 5-amino-2,3-dihydro-1,4-phthalazinedione sodium salt is nearly as effective as prednisolone and much more effective than deflazacort.

5-amino-2,3-dihydro-1,4-phthalazinedione (luminol) belongs to the pharmaceutical class of the phthalazinediones. Compounds of this class are known for their beneficial anti-inflammatory action. 5-amino-2,3-dihydro-1,4-phthalazinedione is also known under the name luminol. Luminol has excellent chemiluminescent properties. It is widely applied in diagnostic assays as a detection means and in forensic medicine, for example for tracing blood spots. In medicine, 5-amino-2,3-dihydro-1,4-phthalazinedione has been developed in the form of a sodium salt. In some countries it is approved for a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione sodium salt was allegedly tested or a beneficial use was suggested (cf. WO 2004/041169; WO 2007/018546; WO 2012/127441; WO 2017/202496; WO 2018/082814: a.o.).

While most conventional immunomodulatory drugs show serious adverse reactions, or are at least problematic in long-term treatment, 5-amino-2,3-dihydro-1,4-phthalazinedione and its pharmaceutically acceptable salts are well tolerated and have a high safety margin in respect to administered dosages.

To ensure a better solubility and bioavailability pharmaceutically acceptable salts of 5-amino-2,3-dihydro-1,4-phthalazinedione are used. Sodium, potassium and lithium salts have been described for therapeutic applications (cf. WO 2010/082858). Crystal structures for lithium, sodium, potassium, rubidium and cesium salts were described in Guzei et al. (2013) Journal of Coordination Chemistry 66, 3722-3739. Thus, the present patent application refers also to the use of all pharmaceutically acceptable salts of 5-amino-2,3-dihydro-1,4-phthalazinedione.

In particular, the present application discloses 5-amino-2,3-dihydro-1,4-phthalazinedione or one of its pharmaceutically acceptable salts for use in the treatment of a congenital muscular dystrophy, wherein the pharmaceutically acceptable salt of 5-amino-2,3-dihydro-1,4-phthalazinedione is 5-amino-2,3-dihydro-1,4-phthalazinedione sodium salt.

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione or one of its pharmaceutically acceptable salts may build complexes with suitable ligands. Thus, the present patent application refers also to such complexes. In the scope of the present disclosure all hydrates and solvates shall be included in the term "5-amino-2,3-dihydro-1,4-phthalazinedione or one of its pharmaceutically acceptable salts".

In order to ensure a reproducible and standardized API production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings [Å] and of the corresponding 2-theta (2θ) angles [°] under which Bragg reflections occur. This yields a unique and unambiguous fingerprint of the respective polymorphs.

For Form I the following values were determined:
d values: 13.5; 6.9; 5.2; 4.6; 3.9; 3.5; 3.4; 3.3; 3.1; 3.0 and/or
2-theta values: 6.5; 12.7; 16.9; 19.3; 22.8; 25.8; 26.6; 27.2; 28.7; 30.3.

Form II is characterized by the following values:
d values: 12.9; 7.9; 7.1; 6.5; 5.3; 4.0; 3.7; 3.6; 3.3; 3.2 and/or
2-theta values: 6.8; 11.2; 12.5; 13.7; 16.7; 22.4; 24.3; 24.9; 27.2; 27.8.

Form III yielded the following values:
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93.

The use of anhydrous Form I of 5-amino-2,3-dihydro-1,4-phthalazinedione sodium salt is preferred.

5-amino-2,3-dihydro-1,4-phthalazinedione itself shows also polymorphism. A Form I (Paradies (1992) Ber. Bunsen-Ges. Phys. Chem 96: 1027-1031) and a Form II (WO 2017/140430) have been disclosed.

Thus, the present patent application refers also to the use according to the disclosure of all crystalline forms and polymorphs thereof of 5-amino-2,3-dihydro-1,4-phthalazinedione and its pharmaceutically acceptable salts. The use of Form II of 5-amino-2,3-dihydro-1,4-phthalazinedione is preferred.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the disclosure.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione (Proescher and Moody (1939) J Lab Clin Med, 1183-1189). Thus, the present patent application refers also to the use of all tautomers of 5-amino-2,3-dihydro-1,4-phthalazinedione and its pharmaceutically acceptable salts.

As used throughout the present application the term "5-amino-2,3-dihydro-1,4-phthalazinedione or one of its pharmaceutically acceptable salts" shall encompass all beforementioned molecular variants of 5-amino-2,3-dihydro-1,4-phthalazinedione, i.e., 5-amino-2,3-dihydro-1,4-phthalazinedione or one of its pharmaceutically acceptable salts or solvates, hydrates, crystalline polymorphs or tautomers thereof.

Unless otherwise stated, any technical or scientific term used in the present invention has the meaning that a man skilled in the relevant technical art will attribute to them.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" in regard to the substance or the pharmaceutical combination of the disclosure or any other active substance mentioned in the description refers to causally occurring beneficial consequences in the organism to which said substance has been administered before.

According to the disclosure the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially improve the disease course of a congenital muscular dystrophy by stopping or decelerating the increase of disabilities during the course of the disease.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to a congenital muscular dystrophy. It refers in particular to medical conditions of a patient in which the manifestation of such symptoms is expected to occur in the far or near future with a reasonable probability.

The terms "subject" and "patient" comprise individuals suffering from disease symptoms or disabilities related to a congenital muscular dystrophy wherein said diagnosis is either approved or suspected. Individuals are mammals, in particular humans.

The term "muscular dystrophies" is not always precisely used in scientific and popular literature. Sometimes it refers also to general conditions of a wasting of muscle mass or massive weight loss, as e.g., sarcopenia, cachexia after cancer, chemotherapy or excessive fasting, dexamethasone-induced muscle wasting or age-related muscle wasting (for example in Sakuma et al. (2014) Front Aging Neurosci 6: 230; Saimithra et al. (2018) http://nopr.niscpr.res.in/handle/123456789/44283 ). These conditions should be better termed "muscle atrophies". The present disclosure relates only to muscular dystrophies in a stricter sense. To avoid any ambiguities, the term "congenital muscular dystrophies" is used throughout the present disclosure.

Congenital muscular dystrophies (CMD) comprise a heterogenous group of disorders that disrupt the function of different muscles or muscle groups. CMD become manifest with progressive muscle weakness, muscle atrophy, spasm, muscle hypertonia, and myalgias. Many CMD involve cardiac failure and respiratory dysfunction, leading to premature death. Over 800 monogenic mutations have been associated with human CMD. Most of them are associated with genes encoding for structural muscle proteins (Chemello et al. (2020) J Clin Invest 130: 2766-2776).

Duchenne muscular dystrophy (DMD) is the most common muscular hereditary disease. Prevalence ranges from 1:3,600 to 1:6,000. Because of the X-linked recessive inheritance nearly exclusively boys are affected. The onset of muscle weakness starts around the age of four. Disease progression is quick. Muscle loss typically occurs first in the thighs and pelvis followed by the arms. Most are unable to walk by the age of 12. Due to muscle wasting a painful malposition of the articulations and bone deformation occur. With adequate disease management patients can reach their fourth decade. At a late stage severe cardiovascular and respiratory problems will finally lead to death.

The disease is caused by a mutation in the gene for the protein dystrophin. Dystrophin is important to maintain the muscle fiber's cell membrane by linking the cell membrane with the actin filaments of the cytoskeleton. Cellular pathophysiology includes mitochondrial dysfunction, reduced ATP levels and an increased Ca²⁺ influx due to the damaged sarcolemma. Damaged mitochondria are not able anymore to buffer intracellular Ca²⁺ surges. As a compensatory mechanism, pAMPK (phosphorylated AMP-activated protein kinase) is increased as well as the number of slow fibers and utrophin. Down-regulation of nNOS (neuronal NO synthase) leads to a lack of nitric oxide. This may lead to local ischemic events due to a lack of vasodilation (cf. Heydemann (2018) Nutrients 10: 796).

Until now, there is no working causal therapy for DMD. An approach of gene therapy by which a functional dystrophin gene should be introduced into the genome was only moderately successful, as autoimmune reactions occurred. Experiments with CRISPR/Cas gene scissors are still at a very early stage. Moderate clinical improvement could be observed by exon-skipping with ataluren. Antisense oligonucleotides skip the defect part (here, a stop codon) of the dystrophin gene. The product is a truncated but partially functional dystrophin (cf. Dunckley et al. (1998) Human Mol Gen 7: 1083-1090). Until now, a periodic redelivery to the affected muscles is needed which is cumbersome and stressful for the patients.

Pharmacologic approaches for a symptomatic treatment are above all based on glucocorticoids. Prednisone, prednisolone and deflazacort are commonly prescribed. The new synthetic glucocorticoid vamorolone is still under development for DMD. The objective is to retard muscle degeneration. β₂ agonists such as salbutamol are used as well as anticonvulsants to control seizures and immunosuppressants. Still in an experimental phase are Na⁺/Ca²⁺ exchange pump inhibitors, NF-κB inhibitors, antioxidants, mitochondrial dysfunction modulators, HDAC (histone deacetylase) inhibitors, anti-fibrotic agents, myostatin inhibitors, TGF-β pathway anti-fibrotic agents and PDE5 inhibitors against muscle ischemia (cf. Guiraud and Davies (2017) Current Opinion in Pharmacology 34: 36-48).

A CMD similar to DMD is Becker muscular dystrophy (BMD; synonym: Becker-Kiener muscular dystrophy). It is likewise an inherited disorder of the dystrophin gene. The associated point mutation in the dystrophin gene affords for the generation of a truncated protein, but in contrast to DMD, this truncated dystrophin is still partially working. Therefore, the disease course is milder than in DMD. Patients can often still maintain an active lifestyle. The prevalence of BMD is approximately 1.5 to 6 in 100,000 male births and thus less frequent than DMD. Symptoms usually appear around ages 8 to 25. In unfortunate disease courses patients may die at age of 40, others reach a normal age. Symptoms include typically progressive muscle weakness of the legs and the pelvis, leading to a gradual difficulty with walking. Further symptoms are upper extremity muscle weakness, toe-walking, breathing difficulties, skeletal deformities such as scoliosis, pseudohypertrophy of calf muscles, muscle cramps, heart muscle problems and elevated creatine kinase levels in blood (htpps://patient.info/doctor/beckers-muscular dystrophy, as of September 12^{th}, 2021). There is no known cure for BMD. Physical therapy may help to mitigate the symptoms. The glucocorticoid prednisone is administered for increasing the production of the dystrophin-related protein utrophin. Because of the comparable etiology the same medications are used as in DMD.

The group of limb girdle dystrophies are associated with point mutations in the autosomally located genes encoding for α-, β-, γ-, δ- and ε-sarcoglycanes. Sarcoglycanes are transmembrane proteins involved in the protein complex responsible for connecting the muscle fiber skeleton to the extracellular matrix. The age of onset us usually 10 to 30 years of age. Both genders are affected equally. Prevalence is about 1 : 14,500. A progressive muscle wasting occurs that affects predominantly hip and shoulder muscles. Symptoms include great difficulties in walking, bending over and squatting, Further symptoms are pseudohypertrophy, muscle hypertrophy, respiratory muscle problems, palpitation, distal muscle problems, facial muscle weakness and weak shoulder muscles. Normally, limb girdle dystrophies do not have a fatal course. Physical therapy may help to alleviate the symptoms. Until now there is no pharmaceutical therapy. However, antioxidant therapy is suggested.

The group of dysferlinopathies encompasses myopathies caused by a mutation in the gene DYSF which encodes for dysferlin, a protein linked with skeletal muscle repair. Examples for dysferlinopathies include Miyoshi myopathy, limb girdle muscular dystrophy type 2B and distal myopathy. These diseases become usually apparent in the third of fourth decade of life. Typical symptoms are weakness and wasting of voluntary skeletal muscles such as the gastrocnemius muscle and the anterior tibial muscles. A focus of prevalence is the Middle East and the Indian subcontinent. Until now, no curative treatment exists. As therapies, synthesis of functional protein after adeno-associated virus vector transfer, gene surgery (exon skipping, trans-splicing) and pharmacological and immunological approaches are discussed. The administration of dantrolene did not turn out to improve the phenotype. Clinical trials with intravascular immunoglobulins (IV-IG) were not pursued. A candidate substance is rituximab, a monoclonal antibody directed against CD20-positive B cells (cf. Barthélémy et al. (2011) Mol Med 17: 875-882). Co-enzyme Q₁₀ and resveratrol showed promising results in mice.

The group of GNE (bifunctional UDP-*N*-acetylglucosamine 2-epimerase / *N-*acetylmannosamine kinase) myopathies includes hereditary inclusion body myopathy (HIBM), distal myopathy with rimmed vacuoles (DMRV), Nonaka distal myopathy and quadriceps-sparing myopathy. These mutations are most frequent in persons of Iranian Jewish descent. Muscle wasting starts around the age of 20 to 30 years, with a disease course of 10 to 15 years to disability. Symptoms include difficulty in walking on heels and in running, a weak index finger, frequent loss of balance. Until now, there is only palliative treatment, e.g. with ankle foot orthoses.

The group of spinal and bulbar muscular atrophies (SMA) encompasses SMARD1, Werdnig-Hoffmann disease and Kugelberg-Welander disease. These disorders are characterized by a degeneration of motoneurons in the anterior horn. This is due to a mutation in the SMN1 gene that encodes for the so-called survival of motoneuron protein. It is inherited in an autosomal recessive manner. Symptoms include areflexia, particularly in the extremities, general muscle weakness, poor muscle tone, difficulties in sitting, standing and walking, weak cough and weak cry in infants, a bell-shaped torso, fasciculation of the tongue and sucking and swallowing difficulties. Pharmaceutical treatment attempts include nusinersen, an antisense nucleotide that modifies the alternative splicing of the adjuvant SMN2 gene, transgenic treatment with onasemnogene as well as risdiplam, a pyridazine derivative that also modifies the SMN2 splicing pattern. If left untreated, most infant patients do not reach the age of 4. This disease is the major course of infant death due to a genetic cause. As there is no adequate pharmaceutical treatment, SMA patients tend to deteriorate over time. The pathophysiology includes oxidative stress. In a transgenic mouse model, the curcumin derivative ASC-JM17, a Nrf2 activator, showed to be beneficial.

Myotonic dystrophy is an autosomal hereditary disease that causes progressive muscle loss and wasting. Muscles are often unable to relax after contraction. Other common symptoms are cataracts, intellectual disability and heart conduction problems Prevalence is about 1:8,000 worldwide. The age of onset is typically in the 20s and 30s. The disease is caused by a mutation in the DMPK gene encoding for myotonin-protein kinase (type 1, DM1) or by a mutation is the CNBP gene encoding for cellular nuclear acid-binding protein (type 2, DM2). Currently, there is no cure for the disease. Treatments include braces or wheelchairs, pacemakers and non-invasive positive pressure ventilation. Mexiletine or carbamazepine are prescribed for muscle relaxation. Pain may be treated with tricyclic antidepressants and NSAIDs (non-steroidal anti-inflammatory drugs) (cf. Meola and Cardani (2015) Biochim Biophys Acta 1852: 594-606).

Emery-Dreyfuss muscular dystrophy (EMD) is caused by a mutation in the gene encoding for emerin. There are types EDMD1 - EDMD7. Symptoms include muscle weakness, above all in the shoulders and lower legs, shortening of the Achilles tendon and the elbow muscles, bradycardia, palpitations, contractures of the muscles. In the course of the disease patients may need orthopedics (walker, cane), physical therapy and respiratory aid. Pharmaceutical treatment is based on beta-blockers and ACE inhibitors.

Facioscapulohumeral muscular dystrophy (FSHD) is caused by a mutation leading to a dysregulation of the gene encoding for double homeobox 4 (DUX4). In this disease, preferentially the skeletal muscles of the face, those that position the scapula and those in the upper arm, overlying the humerus are impaired (cf. Wagner (2019) Lifelong Learning in Neurology 25: 1662-1681). Prevalence ranges between 1:8,000 and 1:20,000. The disease typically becomes manifest between 15 and 30 years of age. Life expectancy is normally not reduced. No therapy could considerably slow down the progress of the disease until now. Treatment includes physical and occupational therapy as well as reconstructive surgery, if needed. Pharmaceutical treatment focuses on the glucocorticoid prednisone, the β₂ agonist albuterol, the calcium channel blocker diltiazem, stamulumab (an antibody inhibiting myostatin) and the TGF-β inhibitor ACE-083.

Oculopharyngeal muscular dystrophy (OPMD) is characterized by an autosomal mutation in the gene encoding for polyadenylate-binding protein 2 (PABPN1). The onset is relatively late in life (40 - 50 years of age). Symptoms include ptosis, weakness of extraocular muscles, dysphagia, aspiration pneumonia and proximal limb weakness. Currently, no therapy exists for stopping disease progress. Treatment attempts target specific diets and cricopharyngeal myotomy to mitigate the symptoms.

Myofibrillar myopathies (types 1 - 6, MFM1-6) are a group of clinically and genetically heterogeneous diseases of the skeletal musculature with an age of onset after 40 years of age. Clinically, slowly progressive muscle weakness with varying distribution of pareses is typical; a differentiation is made between distal, scapuloperoneal, and limb-girdle phenotypes, potentially accompanied by cardiomyopathy, pulmonary involvement, and peripheral neuropathy. Morphology reveals dissolution of myofibrils, deposition of myofibrillar degradation products, abnormal expression of numerous intramuscular proteins, and intracellular desmin-positive protein aggregates. If the heart problems and breathing problems are managed, people with MFM are expected to have a normal life expectancy. Affected genes are DES (desmin), *MYOT* (myotilin), *LDB3* (LIM-domain binding 3, ZASP), *FLNC* (filamin C), *CRYAB* (alpha-crystalline B), *BAG3* (BAG family molecular chaperone regulator 3), *FHL1* (four and a half LIM domain protein 1) and *DNAJB6* (DNaJ homolog subfamily B member 6). Until now there is no causal therapy for myofibrillar myopathies. If necessary, symptomatic treatment e.g. for the cardiovascular problems is used.

Further CMD encompass
- Walker-Warburg syndrome (mutation in the gene for O-mannosyltransferase 1; POMT1, POMT2, FCMD, FKRP),
- Lamin A/C-related congenital muscular dystrophy (mutation in the gene for lamin A/C; LMNA),
- Fukuyama congenital muscular dystrophy (mutation in the gene for fukutin; FCMD),
- Congenital muscular dystrophy with partial merosin deficiency,
- Rigid spine muscular dystrophy (mutation in the gene for selenoprotein N; SEPN 1),
- Congenital muscular dystrophy with primary laminin 2 deficiency (mutation in the gene for laminin α2; LAMA2),
- LARGE-related congenital muscular dystrophy (mutation in the gene for acetylglucosaminyltransferase-like protein; LARGE),
- Muscle-eye-brain disease (mutation in the gene for protein-O-linked mannose β1; POMGnT1),
- Ullrich congenital muscular dystrophy (= Bethlem myopathy), (mutation in the gene for collagen VI; COL6A1-3), and
- Integrin α7 dystrophy (ITGA7).

Thus, the present application refers to 5-amino-2,3-dihydro-1,4-phthalazinedione or one of its pharmaceutically acceptable salts for use in the treatment of a congenital muscular dystrophy, in particular of Duchenne muscular dystrophy or Becker muscular dystrophy.

In particular, the present application refers to 5-amino-2,3-dihydro-1,4-phthalazinedione sodium salt for use in the treatment of a congenital muscular dystrophy, in particular of Duchenne muscular dystrophy or Becker muscular dystrophy.

In another embodiment of the invention, the present application refers to 5-amino-2,3-dihydro-1,4-phthalazinedione or one of its pharmaceutically acceptable salts for use in the treatment of a congenital muscular dystrophy that is selected from a group consisting of limb girdle dystrophies, dysferlinopathies, GNE myopathies, spinal and bulbar muscular atrophies, myotonic dystrophy, Emery-Dreyfuss muscular dystrophy, facioscapulohumeral muscular dystrophy, oculopharyngeal muscular dystrophy, myofibrillar myopathies, Walker-Warburg syndrome, lamin A/C-related congenital muscular dystrophy, Fukuyama congenital muscular dystrophy, congenital muscular dystrophy with partial merosin deficiency, rigid spine muscular dystrophy, congenital muscular dystrophy with primary laminin 2 deficiency, LARGE-related congenital muscular dystrophy, muscle-eye-brain disease, Ullrich congenital muscular dystrophy, and integrin α7 dystrophy.

In another aspect, not claimed, of the disclosure the present application refers thus also to a pharmaceutical composition consisting of 5-amino-2,3-dihydro-1,4-phthalazinedione or one of its pharmaceutically acceptable salts and at least one glucocorticoid for use in the treatment of a congenital muscular dystrophy, in particular of Duchenne muscle dystrophy or Becker muscle dystrophy.

In particular, in another aspect, not claimed, of the disclosure, the present application refers thus to a pharmaceutical composition consisting of 5-amino-2,3-dihydro-1,4-phthalazinedione sodium salt and at least one glucocorticoid for use in the treatment of a congenital muscular dystrophy, in particular of Duchenne muscle dystrophy or Becker muscle dystrophy.

In another aspect, not claimed, of the disclosure, the present application refers to a pharmaceutical composition consisting of 5-amino-2,3-dihydro-1,4-phthalazinedione or one of its pharmaceutically acceptable salts and at least one glucocorticoid for use in the treatment of a congenital muscular dystrophy that is selected from a group consisting of limb girdle dystrophies, dysferlinopathies, GNE myopathies, spinal and bulbar muscular atrophies, myotonic dystrophy, Emery-Dreyfuss muscular dystrophy, facioscapulohumeral muscular dystrophy, oculopharyngeal muscular dystrophy, myofibrillar myopathies, Walker-Warburg syndrome, lamin A/C-related congenital muscular dystrophy, Fukuyama congenital muscular dystrophy, congenital muscular dystrophy with partial merosin deficiency, rigid spine muscular dystrophy, congenital muscular dystrophy with primary laminin 2 deficiency, LARGE-related congenital muscular dystrophy, muscle-eye-brain disease, Ullrich congenital muscular dystrophy, and integrin α7 dystrophy. The use of 5-amino-2,3-dihydro-1,4-phthalazinedione in this pharmaceutical composition is preferred.

Suitable for such a pharmaceutical composition are glucocorticoids selected from a group comprising prednisone, prednisolone, deflazacort, vamorolone, flumethasone, triamcinolone acetonide, betamethasone, dexamethasone, beclomethasone, betamethasone valerate, betamethasone dipropionate, budesonide, beclomethasone dipropionate, isoflupredone, fluocinonide, fluocinolone, methylprednisolone, halcinonide, desonide, deltasone, triamcinolone, triamcinolone acetonide, tixocortol pivalate, mometasone furoate, amcinonide, fluocortolone, halometasone, alclometasone dipropionate, prednicarbate, clobetasone-17-butyrate, clobetasol-17-propionate, fluocortolone caproate, fluocortolone pivalate, fluprednidene acetate, ciclesonide, flunisolide, fluticasone furoate, fluticasone propionate, cortisol, hydrocortisone, hydrocortisone acetate, hydrocortisone-17-valerate, hydrocortisone-17-butyrate, hydrocortisone-17-aceponate, hydrocortisone-17-buteprate, cortisone and cortisone acetate.

Preferred are prednisone, prednisolone, deflazacort and vamorolone.

In another aspect, not claimed, of the disclosure the present application refers to a pharmaceutical composition containing 5-amino-2,3-dihydro-1,4-phthalazinedione or one of its pharmaceutically acceptable salts or the pharmaceutical combination as defined before and at least one pharmaceutically acceptable excipient for use in the treatment of congenital muscular dystrophies.

Preferred is this pharmaceutical composition for use in the treatment of Duchenne muscular dystrophy or Becker muscular dystrophy.

The term "pharmaceutically acceptable excipient(s)" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the disclosure include, carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents (antiadherents), sorbents, permeation enhancers, foaming agents, anti-foaming agents, opacifiers, fatliquors, consistency enhancers, hydrotropes, aromatic and flavoring substances.

In general, one or more pharmaceutically acceptable carriers are added to a pharmaceutically active agent. Eligible are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylglycols, fatty acid esters of sorbitan. Suspensions according to the disclosure may use carriers known in the art such as diluents (e.g., water, ethanol or propylene glycol), ethoxylated isostearyl alcohols, polyoxyethylene and polyoxyethylene sorbitan esters, microcrystalline cellulose, bentonites, agar agar, tragacanth.

The term binding agents refers to substances that bind powders or glue them together, rendering them cohesive through granule formation. They serve as a "glue" of the formulation. Binding agents increase the cohesive strength of the provided diluent or filler.

Suitable binding agents are for example starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indanthrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(*N*-morpholino) ethanesulfonic acid), maleic acid, cacodylate (dimethyl arsenate), carbonic acid, ADA (*N-*(2-acetamido)imino diacetic acid, PIPES (4-piperazine-bis-ethanesulfonic acid), BIS-TRIS propane (1,3-bis[tris(hydroxymethyl)methylamino] propane), ethylene diamine, ACES (2-[(amino-2-oxoethyl)amino]ethanesulfonic acid), imidazole, MOPS (3-(*N*-morpholino) propanesulfonic acid), diethyl malonic acid, TES (2-[tris(hydroxymethyl)methyl]aminoethanesulfonic acid), HEPES (*N*-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), MOPS and *N,N*-bis-(2-hydroxyethyl)-2-aminoethanesulfonic acid (BES). Another group of preferred buffers are glycine, glycyl-glycine, glycyl-glycyl-glycine, *N,N*-bis-(2-hydroxyethyl)glycine and *N*-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N-*trimethyllysine, 3-methyl histidine, 5-hydroxy lysine, o-phosphoserine, gamma-carboxyglutamate, [epsilon]-*N*-acetyl lysine, [omega]-*N*-methyl arginine, citrulline, ornithine and their derivatives. Particularly preferred is KH₂PO₄ buffer.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of a sufficient amount of antioxidants is particularly preferable for liquid and topical dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of sodium metabisulfite, alpha-tocopherol and ascorbyl palmitate.

Tablets or pills are usually coated i.e., the coating constitutes the outer layer. This can be a film coating, a sugar coating with saccharides and a compression coating. Pharmaceutically acceptable varnishes or waxes, HPMC (hydroxypropylmethylcellulose), MC (methylcellulose) or HPC (hydroxypropylcellulose) can be used. Such a coating may help to disguise the taste, to ease the swallowing or the identification. Often plasticizers and pigments are included in the coating. Capsules normally have a gelatinous envelope that encloses the active substance(s). The specific composition and thickness of this gelatinous layer determines how fast absorption takes place after ingestion of the capsule. Of special interest are sustained release formulations, as known in the art.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising, but not limited to, polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzyme-treated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxypropyl starch, hydroxy propyl distarch glycerin, hydroxypropyl distarch phosphate, hydroxypropyl distarch glycerol, starch sodium octenyl succinate, acetylated oxidized starch, hydroxyethyl cellulose.

Suitable pH-regulators for liquid dosage forms are e.g., sodium hydroxide, hydrochloric acid, buffer substances such as sodium dihydrogen phosphate or disodium hydrogenphosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are ammonium chloride and calcium chloride.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions a.o. Moisture expansion is supported by for example starch, cellulose derivatives, alginates, polysaccharides, dextrans, cross-linked polyvinyl pyrrolidone. The amount of the disintegrant in the composition may vary between 1 and 40% per weight, preferred between 3 and 20% per weight, most preferred between 5 and 10% per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10% per weight, preferred between 0.1 and 7% per weight, more preferred between 0.2 and 5% per weight, most preferred between 0.5 and 2% per weight.

The term "lubricants" refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15% per weight, preferred between 0.2 and 5% per weight, more preferred between 0.3 and 3% per weight, most preferred between 0.3 and 1.5% per weight. Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed Eucheuma seaweed, locust bean gum, tragacanth, acacia gum, karaya gum, gellan gum, gum ghatti, glucomannan, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Suitable as surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates.

Stabilizers are substances that can be added to prevent unwanted changes. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs for handling minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthan gum, gum arabic or any combination thereof.

Anti-caking agents (antiadherents) can be added to a supplement or a composition of supplements for preventing the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

Permeation enhancers are often used in topical dosage forms. Suitable permeation enhancers comprise all pharmaceutically acceptable permeation enhancers known in the art, such as, without being limiting, azones such as laurocapram, 1-dodecylazacycloheptan-2-one; sulfoxides such as dimethyl sulfoxide, DMAC, DMF; pyrrolidones such as 2-pyrrolidone, N-methyl-2-pyrrolidone; alcohols such as ethanol, 1,2-propandiol or decanol; glycols such as propylene glycol, diethylene glycol, tetraethylene glycol; fatty acids such as oleic acid, lauric acid, sodium lauryl sulfate, myristic acid, isopropyl myristic acid, capric acid; nonic surfactants such as polyoxyethylene-2-oleyl ether, polyoxyethylene-2-stearyl ether; terpenes; terpenoids; oxazolidinones; urea; ceramide analogs, azone analogs, menthol derivatives, etherified derivatives, esterified derivatives, transcarbams, carbamate salts, TXA derivatives, DDAIP (dodecyl 2-(dimethylamino) propanoate), DDAK, natural essential oils (all of them listed in Chen et al. (2014) Asian J. Pharm. Sc. 9, 51-64); citric acid esters such as triethyl citrate; hydrophobin polypeptides; alpha-bisabolol; dimethyl isosorbide (Arlasove^{®} DMI); ethoxydiglycol. Preferred is 1,2-propandiol.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer). Examples are polydimethylsiloxane or silicone oil in dietary supplements or simethicone in pharmaceuticals.

Opacifiers are substances that render the liquid dosage for, opaque, if desired. They must have a refractive index substantially different from the solvent, in most cases here water. At the same time, they should be inert to the other components of the composition. Suitable examples include titanium dioxide, talc, calcium carbonate, behenic acid, cetyl alcohol, or mixtures thereof.

Suitable fatliquors are e.g., oleic acid decyl ester, hydrated castor oil, light mineral oil, mineral oil, polyethylene glycol, sodium laurylsulfate.

Consistency enhancers are e.g., cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from achillea, sage, cedar, clove, chamomile, anise, aniseed, star anise, thyme, tea tree, peppermint, mint oil, menthol, cineol, borneol, zingerol, eucalyptus, mango, figs, lavender oil, chamomile blossoms, pine needle, cypress, orange, rose, rosewood, plum, currant, cherry, birch leaves, cinnamon, lime, grapefruit, tangerine, juniper, valerian, lemon, lemon balm, lemon grass, palmarosa, cranberry, pomegranate, rosemary, ginger, pineapple, guava, echinacea, ivy leave extract, blueberry, kaki, melon, alpha- or beta-pinene, alpha-pinene oxide, alpha-campholenic aldehyde, alpha-citronellol, alpha-isoamyl-cinnamic, alpha-cinnamic terpinene, alpha-terpineol, alpha-terpinene, aldehyde C₁₆, alpha-phellandrene, amyl cinnamic aldehyde, amyl salicylate, anisic aldehyde, basil, anethole, bay, benzyl acetate, benzyl alcohol, bergamot, bitter orange peel, black pepper, calamus, camphor, cananga oil, cardamom, carnation, carvacrol, carveol, cassia, castor, cedarwood, cinnamaldehyde, cinnamic alcohol, cis-pinane, citral, citronella, citronellal, citronellol dextro, citronellol, citronellyl acetate; citronellyl nitrile, citrus unshiu, clary sage, clove bud, coriander, corn, cotton seed, d-dihydrocarvone, decyl aldehyde, diethyl phthalate, dihydroanethole, dihydrocarveol, dihydrolinalool, dihydromyrcene, dihydromyrcenol, dihydromyrcenyl acetate; dihydroterpineol, dimethyl salicylate, dimethyloctanal, dimethyloctanol, dimethyloctanyl acetate, diphenyl oxide, dipropylene glycol, d-limonen, d-pulegone, estragole, ethyl vanillin, eucalyptol; eucalyptus citriodora, eucalyptus globulus, eugenol, evening primrose, fenchol, fennel, ferniol, fish, florazon, galaxolide, geraniol, geranium, geranyl acetate, geranyl nitrile, guaiacol, guaiacwood, gurjun balsam, heliotropin, herbanate, hiba, hydroxycitronellal, i-carvone, i-methyl acetate, ionone, isobutyl quinoleine, isobornyl acetate, isobornyl methylether, isoeugenol, isolongifolene, jasmine, lavender, limonen, linallol oxide, linallol, linalool, linalyl acetate, linseed, litsea cubeba, I-methyl acetate, longifolene, mandarin, mentha, menthane hydroperoxide, menthol crystals, menthol laevo, menthone laevo, methyl anthranilate, methyl cedryl ketone, methyl chavicol, methyl hexyl ether, methyl ionone, methyl salicylate, mineral, mint, musk ambrette, musk ketone, musk xylol, myrcene, nerol, neryl acetate, nonyl aldehyde, nutmeg, orris root, para-cymene, parahydroxy phenyl butanone crystals, patchouli, p-cymene, pennyroyal oil, pepper, perillaldehyde, petitgrain, phenyl ethyl alcohol, phenyl ethyl propionate, phenyl ethyl-2methylbutyrate, pimento berry, pimento leaf, pinane hydroperoxide, pinanol, pine ester, pine, pinene, piperonal, piperonyl acetate, piperonyl alcohol, plinol, plinyl acetate, pseudo ionone, rhodinol, rhodinyl acetate, rosalin, ryu, sandalwood, sandenol, sassafras, sesame, soybean, spearmint, spice, spike lavender, spirantol, starflower, tea seed, terpenoid, terpineol, terpinolene, terpinyl acetate, tert-butylcyclohexyl acetate, tetrahydrolinalool, tetrahydrolinalyl acetate, tetrahydromyrcenol, thulasi, thymol, tomato, trans-2-hexenol, trans-anethole, turmeric, turpentine, vanillin, vetiver, vitalizair, white cedar, white grapefruit, wintergreen etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

According to the disclosure all the beforementioned excipients and classes of excipients can be used without limitation alone or in any conceivable combination thereof, as long as the inventive use is not thwarted, toxic actions may occur, or respective national legislations are infracted.

In another aspect of the invention the present application relates to 5-amino-2,3-dihydro-1,4-phthalazinedione or one of its pharmaceutically acceptable salts, a pharmaceutical composition according to the disclosure or a pharmaceutical combination according to the disclosure for use in a formulation for oral administration in the treatment of a congenital muscular dystrophy, in particular of Duchenne muscle dystrophy or Becker muscle dystrophy.

Pharmaceutical formulations suitable for oral dosage forms of 5-amino-2,3-dihydro-1,4-phthalazinedione or one of its pharmaceutically acceptable salts, a pharmaceutical composition according to the disclosure or a pharmaceutical combination according to the disclosure may be administered as separate units such as tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agent can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner e.g., an edible carbohydrate such as starch or mannitol. A flavor, preservative, dispersant or colorant can also be present.

Tablets are formulated by producing, granulating or dry pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the disclosure can also be combined with a free-flowing inert excipient and then pressed directly to give tablets without carrying out the granulation or dry-pressing steps.

In another aspect of the invention 5-amino-2,3-dihydro-1,4-phthalazinedione or one of its pharmaceutically acceptable salts, a pharmaceutical composition according to the disclosure or a pharmaceutical combination according to the disclosure are provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise for improving the availability of the medication after intake of the capsule. Additionally, suitable binding agents and/or colorants can be added to the mixture, if desirable or necessary.

In another aspect of the invention 5-amino-2,3-dihydro-1,4-phthalazinedione or one of its pharmaceutically acceptable salts, a pharmaceutical composition according to the disclosure or a pharmaceutical combination according to the disclosure are included in soft gelatin capsules (SGC). SGCs are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGCs are particularly useful for the administration of poorly water-soluble active agents.

In another aspect of the invention 5-amino-2,3-dihydro-1,4-phthalazinedione or one of its pharmaceutically acceptable salts, a pharmaceutical composition according to the disclosure or a pharmaceutical combination according to the disclosure are included in chewable tablets or hard caramels. Herein the substance is integrated into the matrix of the tablets or caramels.

In another aspect of the invention the present application relates to 5-amino-2,3-dihydro-1,4-phthalazinedione or one of its pharmaceutically acceptable salts, a pharmaceutical composition according to the disclosure or a pharmaceutical combination according to the disclosure for use in the treatment of a congenital muscular dystrophy, in particular of Duchenne muscle dystrophy or Becker muscle dystrophy, in a formulation for inhalatory administration.

For an effective inhalatory treatment of a congenital muscular dystrophy that at one stage of the disease course affects the respiratory organs it is advantageous that 5-amino-2,3-dihydro-1,4-phthalazinedione or one of its pharmaceutically acceptable salts, a pharmaceutical composition according to the disclosure or a pharmaceutical combination according to the disclosure reaches the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers, jet nebulizers, ultrasonic nebulizers, dry powder inhalers and (pressurized) metered-dose inhalers comprise, without being limiting, PARI eFlow^{®}rapid, PARI LC STAR^{®}, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB^{®}-ir, OPTI-NEB^{®}, M-neb^{®} dose⁺ mesh nebulizer inhalation MN-300/8, M-Neb Flow+ and M-neb^{®} mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen^{®} Solo, Aerogen^{®} Ultra and Aerogen^{®} PRO (Aerogen, Galway, Ireland), KTMED NePlus NE-SM1 (KTMED Inc., Seoul, South Korea), Vectura Bayer Breelib^{™} (Bayer AG, Leverkusen, Germany), Vectura Fox, MPV Truma and MicroDrop^{®} Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bia 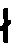 ystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump^{®} KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist^{®} (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus^{®} BBU01 and BBU02 (Tai Yu International Manufactory Ltd., Dongguan, China), TaiDoc TD-7001 (TaiDoc Technology Co., New Taipei City, Taiwan), Vibralung^{®} and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed^{®} JLN-MB001 (Kernmed, Durmersheim, Germany).

Preferred are mesh nebulizers with a piezoelectric activation of the nebulization process, respectively vibrating mesh nebulizers.

Thus, in another aspect of the invention the present application relates to 5-amino-2,3-dihydro-1,4-phthalazinedione or one of its pharmaceutically acceptable salts, a pharmaceutical composition according to the disclosure or a pharmaceutical combination according to the disclosure for use in the treatment of a congenital muscular dystrophy, in particular of Duchenne muscle dystrophy or Becker muscle dystrophy, in a formulation for inhalatory administration, wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers, the nebulized aerosol is continuously released into the mouthpiece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Hcmed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally do not reach the lower airways, they often get stuck in the throat. Particles with a MMAD > 5 µm and < 10 µm usually reach the bronchi but not the alveoli. Particles between 100 nm and 1 µm MMAD do not deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

In another aspect of the invention the present application refers also to a method for producing an aerosol according to the disclosure for the treatment of a congenital muscular dystrophy, comprising the following steps:
a) filling 0.1 ml to 5 ml of an aqueous solution containing 5-amino-2,3-dihydro-1,4-phthalazinedione or one of its pharmaceutically acceptable salts, a pharmaceutical composition according to the disclosure or a pharmaceutical combination according to the disclosure and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz, preferred 90 kHz to 180 kHz, more preferred 100 kHz to 160 kHz, most preferred 105 kHz to 130 kHz (cf. Chen, The Aerosol Society: DDL2019**;** Gardenshire et al. (2017) A Guide to Aerosol Delivery Devices for Respiratory Therapists, 4th ed.).

Thus, the beforementioned method is also disclosed with said vibration frequency ranges.

The method according to the disclosure is thus characterized in that at least 80 % in weight, preferred at least 85 % in weight, most preferred at least 90 % in weight of 5-amino-2,3-dihydro-1,4-phthalazinedione or one of its pharmaceutically acceptable salts, a pharmaceutical composition according to the disclosure or a pharmaceutical combination according to the disclosure contained in said aqueous solution are nebulized in the generated aerosol.

The method of the invention is particularly effective in nebulizing a high percentage of the pharmaceutically active agent(s) from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes' time span 95 % of the substance provided in the aqueous solution could be nebulized. This is an ideal time span for a high patient compliance.

Therefore, the method according to the disclosure is thus characterized in that at least 80 % of the generated aerosol are produced during three minutes after starting nebulization in the mesh nebulizer, preferred at least 85 % and most preferred at least 90 %.

While pharmaceutically active agents are usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another aspect of the invention the present application refers also to a kit comprising a mesh nebulizer and a pharmaceutically acceptable container with an aqueous solution containing an effective amount of 5-amino-2,3-dihydro-1,4-phthalazinedione or one of its pharmaceutically acceptable salts, a pharmaceutical composition according to the disclosure or a pharmaceutical combination according to the disclosure for the treatment of a congenital muscular dystrophy, in particular of Duchenne muscle dystrophy or Becker muscle dystrophy, and optionally at least one pharmaceutically acceptable excipient.

In an alternative kit 5-amino-2,3-dihydro-1,4-phthalazinedione or one of its pharmaceutically acceptable salts, a pharmaceutical composition according to the disclosure or a pharmaceutical combination according to the disclosure is not provided in form of an aqueous solution but in two separated containers, one for a solid form for the active agent and the other for an aqueous solution. The final aqueous solution is freshly prepared by solving the active agent in the final solution. Thereupon the final aqueous solution is filled into the nebulization chamber of the mesh nebulizer. These two containers can be completely separated containers e.g., two vials, or e.g., a dual-chamber vial. For solving the active agent e.g., a membrane between the two chambers is perforated to allow for mixing of the content of both chambers.

Thus, the present application discloses also a kit, comprising a mesh nebulizer, a first pharmaceutically acceptable container with water for injection or physiological saline solution and a second pharmaceutically acceptable container with an effective dosage of a solid form of 5-amino-2,3-dihydro-1,4-phthalazinedione or one of its pharmaceutically acceptable salts, a pharmaceutical composition according to the disclosure or a pharmaceutical combination according to the disclosure for the treatment of a congenital muscular dystrophy, in particular of Duchenne muscle dystrophy or Becker muscle dystrophy, wherein optionally at least one pharmaceutically acceptable excipient is contained in the first pharmaceutically acceptable container and/or the second pharmaceutically acceptable container.

The aerosol generated by the method according to the disclosure is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless-steel injection needle would be in the range of 14 to 27.

In yet another aspect of the invention 5-amino-2,3-dihydro-1,4-phthalazinedione or one of its pharmaceutically acceptable salts, a pharmaceutical composition according to the disclosure or a pharmaceutical combination according to the disclosure for use in the treatment of a congenital muscular dystrophy, in particular of Duchenne muscle dystrophy or Becker muscle dystrophy, wherein said substance, composition or combination is applied in form of liposomes, micelles, multilamellar vesicles or a cyclodextrin complex.

In yet another aspect of the invention, the present application discloses 5-amino-2,3-dihydro-1,4-phthalazinedione or one of its pharmaceutically acceptable salts or a composition according to the invention or a combination according to the invention for use in the treatment a congenital muscular dystrophy, in particular of Duchenne muscle dystrophy or Becker muscle dystrophy, wherein a previous treatment with at least one other pharmaceutically active agent was refractory.

In yet another aspect of the invention, the present application discloses 5-amino-2,3-dihydro-1,4-phthalazinedione or one of its pharmaceutically acceptable salts or a composition according to the invention or a combination according to the invention for use in the treatment a congenital muscular dystrophy, in particular of Duchenne muscle dystrophy or Becker muscle dystrophy, wherein said substance or said pharmaceutical composition is formulated as a suppository.

To produce a dosage form of 5-amino-2,3-dihydro-1,4-phthalazinedione as a suppository, waxes with a low melting point as well as a mixture of fatty acid glycerides such as cocoa butter are first melted, then 5-amino-2,3-dihydro-1,4-phthalazinedione is homogenously dispersed under stirring or other mixing methods. The molten homogeneous mixture is then transferred to suitable molds and cooled down until solidification.

In yet another aspect of the invention the present application relates to 5-amino-2,3-dihydro-1,4-phthalazinedione or one of its pharmaceutically acceptable salts, a pharmaceutical composition according to the disclosure or a pharmaceutical combination according to the disclosure for use in the treatment of a congenital muscular dystrophy, in particular of Duchenne muscle dystrophy or Becker muscle dystrophy, in a liquid dosage form.

The present application also discloses the parenteral administration of 5-amino-2,3-dihydro-1,4-phthalazinedione or one of its pharmaceutically acceptable salts, a pharmaceutical composition according to the disclosure or a pharmaceutical combination according to the disclosure for the treatment of a congenital muscular dystrophy, in particular of Duchenne muscle dystrophy or Becker muscle dystrophy, in the form of intravenous injection, intraarterial injection or intraperitoneal injection.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

A preferred dosage form according to the invention are retard formulations i.e., formulations with a delayed release of the at least one active agent. They are also known as sustained release (*SR*), extended release (*ER, XR*) or controlled/continuous release (*CR*) forms. Suitable formulations and carriers are known to a person skilled in the art (Kleinsorge (1995) Retardformulierungen in der medikamentösen Therapie. Leipzig, Barth 8th ed.). Most commonly, the active agent is embedded in a matrix of insoluble substances like acrylics or chitin. Hence, the active agent must find its way out through orifices in the matrix. In some formulations, there is a laser-drilled hole on one side and opposite to it a porous membrane. The gastric fluid attacks this porous membrane, flows in and pushes the active agent through the drilled hole on the opposite side. In other formulations, the active agent dissolves inside the matrix swelling thereupon and forming a gel. Then the active agent is released through the pores of the gel. Other examples include specifically coated tablets resistant to gastric fluid, retard capsules containing retard pellets of the active agent that are going to be released after the dissolution of the capsule casing, multiple unit pellet systems (MUPS), oral osmotic systems, resonates, coacervation and micro-encapsulation. With the use of such a retard formulation the release site of a drug and its pharmacokinetics can be controlled. For example, it is often desirable that a dosage form of an active agent is not dissolved before reaching a certain point of the intestines. As the pH changes along the way through the intestines, the dissolution process may be engineered to be pH dependent. In therapeutic applications in which the absorption of an active agent through the intestinal mucosa shall be facilitated for increasing its bioavailability it may be preferable not to use a salt of an active agent but its neutral form.

Therefore, the present application refers also to 5-amino-2,3-dihydro-1,4-phthalazinedione or one of its pharmaceutically acceptable salts for use according to the invention or a pharmaceutical composition for use according to the invention, wherein said substance or said pharmaceutical composition are formulated as a retard drug.

In yet another aspect of the invention 5-amino-2,3-dihydro-1,4-phthalazinedione or one of its pharmaceutically acceptable salts, a pharmaceutical composition according to the disclosure or a pharmaceutical combination according to the disclosure for use in the treatment of a congenital muscular dystrophy, in particular of Duchenne muscle dystrophy or Becker muscle dystrophy, wherein said substance, composition or combination is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulas (peripheral venous catheters) and pressure bags.

In general, an aqueous solution or a physiological saline solution is preferred. In case of a poorly soluble pharmaceutical agent according to the disclosure also ethanol or ethanol/water mixtures can be used.

Further suitable liquid dosage forms are drops, gels and hydrogels.

A gel is a colloid in which the solid disperse phase forms a network in combination with that of the fluid continuous phase, resulting in a viscous semirigid sol. Gel properties range from soft and weak to hard and tough. They are defined as a substantially dilute cross-linked system, which exhibits no flow in the steady state. By weight, gels are mostly liquid, yet they behave like solids due to a three-dimensional cross-linked network within the liquid. It is the crosslinking within the fluid that gives a gel its consistency and contributes to the adhesive stick. Gels are a dispersion of molecules of a liquid within a solid medium.

A hydrogel is a network of polymer chains that are hydrophilic, sometimes found as a colloidal gel in which water is the dispersion medium. A three-dimensional solid results from the hydrophilic polymer chains being held together by cross-links. Because of the inherent cross-links, the structural integrity of the hydrogel network does not dissolve from the high concentration of water. Hydrogels are highly absorbent (they can contain over 90% water) natural or synthetic polymeric networks. Hydrogels also possess a degree of flexibility very similar to natural tissue, due to their significant water content. In medicine, hydrogels can encapsulate chemical systems which upon stimulation by external factors such as a change of pH may cause specific pharmaceutically active agent(s) to be liberated to the environment, in most cases by a gel-sol transition to the liquid state.

Suitable gel formers can be selected from the group comprising, but not limited to, agar, algin, alginic acid, bentonite, carbomer, carrageenan, hectorite, hydroxyethyl cellulose, hydroxypropyl cellulose, polyvinyl alcohol, polyvinyl pyrrolidone, sodium carbomer.

In yet another aspect of the invention the present application relates to 5-amino-2,3-dihydro-1,4-phthalazinedione or one of its pharmaceutically acceptable salts, a pharmaceutical composition according to the disclosure or a pharmaceutical combination according to the disclosure for use in the treatment of a congenital muscular dystrophy, in particular of Duchenne muscle dystrophy or Becker muscle dystrophy, in a formulation for sublingual tablets.

Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

In order to avoid a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

When swallowing is avoided, an administration of a pharmaceutically active agent by means of a sublingual tablet can also reach the pharynx/throat topically. Absorption of the pharmaceutically active agent occurs to a good part via the pharyngeal mucosa.

In yet another embodiment of the invention a pharmaceutical composition comprising 5-amino-2,3-dihydro-1,4-phthalazinedione or one of its pharmaceutically acceptable salts for use in the treatment of a congenital muscular dystrophy, in particular of Duchenne muscular dystrophy or Becker muscular dystrophy, is provided as a topical application form, such as creams, emulsions, lotions, gels, hydrogels, pastes, powders, ointments, liniment, films, liposomes, dermal patches, transdermal patches, transdermal sprays or suspensions.

In a further aspect of the invention a method of treatment of a congenital muscular dystrophy, in particular of Duchenne muscle dystrophy or Becker muscle dystrophy, is disclosed, in which an effective dose of 5-amino-2,3-dihydro-1,4-phthalazinedione or one of its pharmaceutically acceptable salts, a pharmaceutical composition according to the disclosure or a pharmaceutical combination according to the disclosure is administered to a patient in need thereof.

The present disclosure refers likewise to 5-amino-2,3-dihydro-1,4-phthalazinedione or one of its pharmaceutically acceptable salts, hydrates or solvates for use in a method for the treatment of a congenital muscular dystrophy, in particular of Duchenne muscle dystrophy or Becker muscle dystrophy, in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of at least one glucocorticoid or one of its pharmaceutically acceptable salts.

The present disclosure refers likewise to 5-amino-2,3-dihydro-1,4-phthalazinedione or one of its pharmaceutically acceptable salts, hydrates or solvates for use in a method for the treatment of a congenital muscular dystrophy, in particular of Duchenne muscle dystrophy or Becker muscle dystrophy, in a subject, wherein the method comprises administering to the subject an affective amount of 5-amino-2,3-dihydro-1,4-phthalazinedione or one of its pharmaceutically acceptable salts, hydrates or solvates and concomitantly or subsequently an effective amount of at least one glucocorticoid or one of its pharmaceutically acceptable salts, wherein 5-amino-2,3-dihydro-1,4-phthalazinedione is a sodium salt.

The present disclosure refers likewise to 5-amino-2,3-dihydro-1,4-phthalazinedione or one of its pharmaceutically acceptable salts, hydrates or solvates for use in a method for the treatment of a congenital muscular dystrophy, in particular of Duchenne muscle dystrophy or Becker muscle dystrophy, in a subject, wherein the method comprises administering to the subject an affective amount of at least one glucocorticoid or one of its pharmaceutically acceptable salts and concomitantly or subsequently an effective amount of 5-amino-2,3-dihydro-1,4-phthalazinedione or one of its pharmaceutically acceptable salts, hydrates or solvates.

The present disclosure refers likewise to 5-amino-2,3-dihydro-1,4-phthalazinedione or one of its pharmaceutically acceptable salts, hydrates or solvates for use in a method for the treatment of a congenital muscular dystrophy, in particular of Duchenne muscle dystrophy or Becker muscle dystrophy, in a subject, wherein the method comprises administering to the subject an affective amount of at least one glucocorticoid or one of its pharmaceutically acceptable salts and concomitantly or subsequently an effective amount of 5-amino-2,3-dihydro-1,4-phthalazinedione or one of its pharmaceutically acceptable salts, hydrates or solvates, wherein 5-amino-2,3-dihydro-1,4-phthalazinedione is a sodium salt.

### EXAMPLES

The *mdx* mouse model is the most widely used and investigated animal model in the research of Duchenne muscular dystrophy since the early 1980ies. These mice are characterized by muscle weakness, histological evidence of myopathy and elevated serum levels of creatin kinase (CK) (cf. Bulfield et al. (1984) PNAS 81: 1189-1192). Herein, CK levels are a marker of the breakdown of CK-rich tissues such as skeletal muscles (rhabdomyolysis). The mutation in the *mdx* mouse is a nonsense mutation (C-to-T transition) in exon 23 that aborts full-length dystrophin expression (cf. Sicinski et al. (1989) Science 244: 1578-1580). Symptoms are moderate and the mean lifespan is reduced by about 25 %. During the first two weeks after birth *mdx* mice are indistinguishable from wild-type mice. Then skeletal muscle necrosis starts until week 6 after birth and thereupon enters in a plateau phase characterized by muscle hypertrophy. Only the diaphragm shows progressive deterioration. Severe dystrophic phenotypes such as muscle wasting, scoliosis or heart failure do not occur until mice are 15 months or older (cf. McGreevy et al. (2015) Dis Model Mech 8: 195 - 213). Many aged *mdx* mice develop spontaneous sarcoma.

Four-week-old C57BL/10ScSnJ (stock #000476) and C57BL/10ScSn-Dmdmdx/J mice (stock #001801) were sourced from Jackson Laboratory (Bar Harbor, USA). Animals were received in 2 cohorts (n=49-56; 7-8 per group). Mice were sorted into a total of five treatment groups. The mice of the control groups were wild type C57BL/10ScSnJ and the dmx strain C57BL/10ScSn-Dmdmdx/J. The animals were acclimatized upon arrival to the animal facility for 7 days, housed in cages with up to 5 mice per cage. During the study, 12-hour light/11-hour dark cycles were maintained. Room temperature was maintained between 20°C and 23°C. Food and water were available *ad libitum* for the duration of the study. Mice were identified by ear tags. Randomization was done based on the average body weight of the cage. These cages were assigned randomly to treatment groups. Animals were monitored daily for changes in behavior. If necessary, a veterinarian decided whether an animal needed to be euthanized to relieve unnecessary pain or distress. In this case it was decided whether a tissue collection was yet to be performed.

The animal experiments were approved by the Dalhouse University Animal Committee in accordance with the Canadian Council on Animal Care.

All treatment compounds were administered via intraperitoneal (IP) injection, with exception of prednisolone that was delivered via oral syrup. IP injections were performed by restraining the mouse and delivering the treatment on either side of the abdomen, halfway between the midline and natural bend of a knee. The needle was inserted at a 45° angle, bevel side up. The syringe was aspirated slightly before the treatment is delivered to ensure a proper intraperitoneal position. Once the injection was delivered, the mouse was monitored for a couple of minutes for any adverse side effects. Animals received the injection on alternating sides daily to reduce damage from repeated injections. The oral syrup containing prednisolone was delivered to each mouse by filling the syringe (without needle tip) with the desired treatment volume. The syringe was checked before administration to ensure that there are no bubbles in the syrup. One the mouse was restrained, the syringe, without tip, was placed at the mouth of the mouse. The syringe plunger was slowly pressed allowing the mouse to lick the syrup. Once the complete treatment was delivered the mouse was placed back into its cage and monitored for a couple of minutes for any adverse side effects.

The experiments were carried out by AGADA Biosciences (Halifax, Canada) on behalf of the applicant. All treatment substances were provided by the applicant. All treatment groups started with 15 mice. Treatment was blinded.

| | strain | treatment [mg/kg body weight] |
|---|---|---|
| group A | wild type (BL10) | vehicle |
| group B | *mdx* | vehicle |
| group C | *mdx* | 1.0 mg/kg 5-amino-2,3-dihydro-1,4-phthalazinedione sodium salt |
| group D | *mdx* | 2.5 mg/kg 5-amino-2,3-dihydro-1,4-phthalazinedione sodium salt |
| group E | *mdx* | 5.0 mg/kg 5-amino-2,3-dihydro-1,4-phthalazinedione sodium salt |
| group F | *mdx* | 1.0 mg/kg prednisolone |
| group G | *mdx* | 1.0 mg/kg deflazacort |

### Example 1: In vitro effects of 5-amino-2,3-dihydro-1,4-phthalazinedione sodium salt on the contractile properties on the musculus extensor digitorum longus muscle in the mdx mouse, in comparison to deflazacort and prednisolone

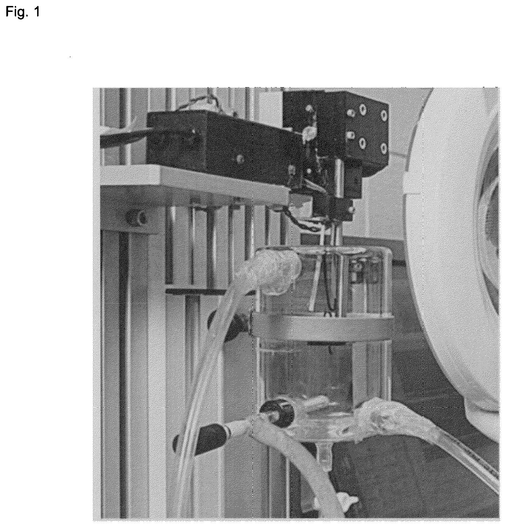

Contractile properties were measured *in vitro* on the right musculus extensor digitorum longus (EDL) at the end of the study. The mice were anesthetized using ketamine and xylamine. The EDL muscle of the right hindlimb was removed from each mouse and immersed in individual carbogenated baths (95% O₂, 5% CO₂) containing Ringer's solution (pH 7.4) at 25°C. The muscle was flanked by two electrodes. Using non-fatiguing twitches, the muscle was maintained at 10 mN for force generation. The maximal force was measured with the muscle held at 10 mN. The muscle was stimulated with an electrode to elicit tetanic contractions that are separated by 2-minute rest intervals. For the EDL with each subsequent tetanus, the stimulation frequency was increased in steps of 20, 30 or 50 Hz until the force reached a plateau which usually occurs around 250 Hz. This plateau was considered the maximum force (mN) generated by the muscle. The cross-sectional area of the muscle was measured based on muscle mass, muscle length, and tissue density. Finally, the muscle specific force (kN/m²) was calculated based on the cross-sectional area of the muscle and was converted in relation to the individual body weight (nM/kg). The experimental set-up is shown in Fig. 1.

In this experiment, 15 mice could be evaluated from treatment groups 1 and 2, 14 mice from groups 3, 4, 6 and 7 and 12 mice from group 5.

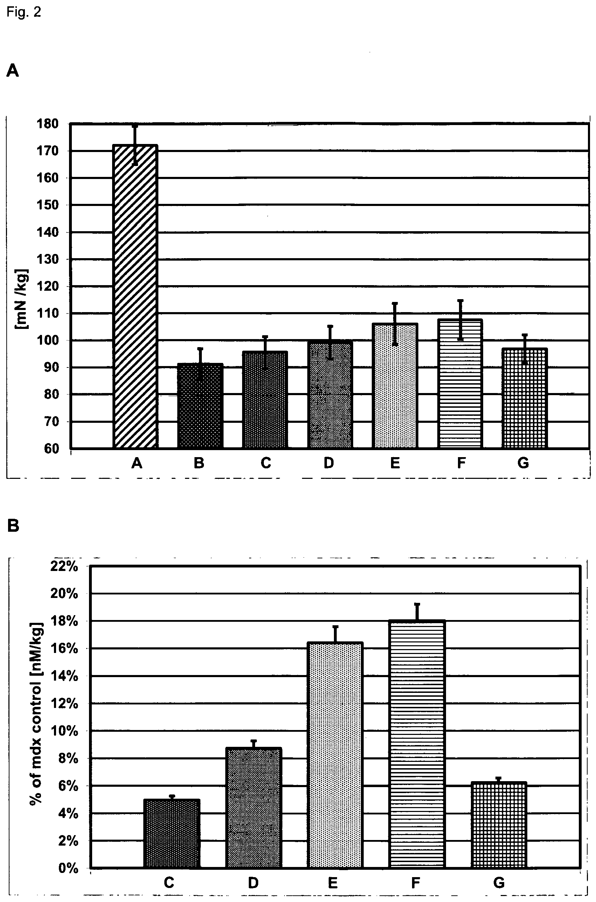

5-amino-2,3-dihydro-1,4-phthalazinedione sodium salt showed a dose-dependent improvement of the maximum force generated by the muscle. The highest dose of 5-amino-2,3-dihydro-1,4-phthalazinedione sodium salt (5.0 mg/kg body weight) is nearly as effective as prednisolone (106.1 ± 8.0 mN/kg vs. 107.5 ± 7.2 mN/kg) but much more effective than deflazacort (96.8 ± 5.2 mN/kg). All data mean ± SEM. In Fig. 2A these data are depicted for all treatment groups. In Fig. 2B the same data are depicted as the percentual improvement of the treatment groups in comparison to the untreated mdx mice control group which was set as 100%. Herein the highest dose of 5-amino-2,3-dihydro-1,4-phthalazinedione sodium salt (5.0 mg/kg body weight) yielded an improvement of 16.4%, in comparison to prednisolone (18.0%) and deflazacort (6.2 %).

These data suggest that 5-amino-2,3-dihydro-1,4-phthalazinedione sodium salt is able to replace glucocorticoid treatment in Duchenne muscle dystrophy and Becker muscle dystrophy patients for obtaining the same beneficial effects but without the adverse side effects of a long-term glucocorticoid treatment. These data also suggest that a combination of 5-amino-2,3-dihydro-1,4-phthalazinedione sodium salt and a glucocorticoid would be beneficial for these patients.

### Example 2: Effects of 5-amino-2,3-dihydro-1,4-phthalazinedione sodium salt treatment on the body weight in the mdx mouse, in comparison to deflazacort and prednisolone

As an adverse side effect, glucocorticoid treatment is known to reduce the body weight of animals in experiments. This is a correlate to the effects of long-term glucocorticoid treatment in Duchenne muscular dystrophy patients (also to some degree in Becker muscular dystrophy patients) that usually develop less muscle mass and show a retarded growth in the course of the long-term treatment.

The body weight was measured weekly starting after acclimation of the animals to the facility and the beginning of the treatment (5 weeks of age). At the start of each week the body weight of each mouse was determined on an OHAUS Scout Pro digital scale that was tared to an open 750 ml Tupperware container. Mice were constrained individually to the container and placed on top of the scale.

As expected, the treatment groups with prednisolone and deflazacort (groups F and G) showed a significantly diminished body weight increase over the treatment, starting at about week 6. Roughly, the difference is about 7%. In the treatment groups with three concentrations of 5-amino-2,3-dihydro-1,4-phthalazinedione sodium salt no differences to the untreated mdx mice and the wild type mice could be observed over the treatment period.

These data suggest that under a treatment with 5-amino-2,3-dihydro-1,4-phthalazinedione sodium salt the usually observed (and feared of) adverse side effect of less muscle mass development and retarded growth is not to be expected. In this aspect 5-amino-2,3-dihydro-1,4-phthalazinedione sodium salt showed to be superior to prednisolone and deflazacort.

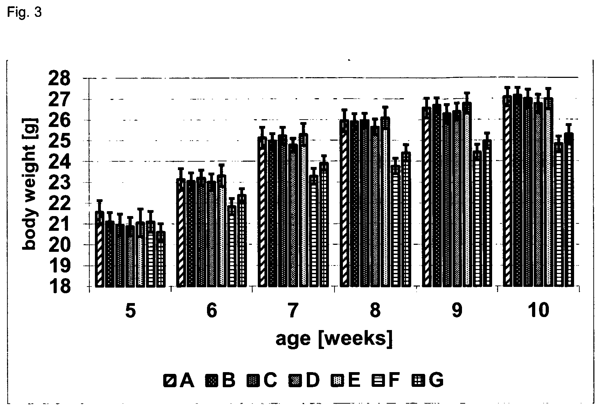

The development of the body weight over the treatment period is depicted for all groups in Fig. 3.

### FIGURES

Fig. 1: Photo of the experimental set-up for the measurement of the contractile properties on the musculus extensor digitorum longus muscle in the mdx mouse as used in Example 1.
Fig. 2:
A: Bar diagram showing the effects of the different treatment groups the measurement of the contractile properties on the musculus extensor digitorum longus muscle in the mdx mouse (Example 1).
B: Bar diagram showing the percentual improvement of the treatment of the different treatment groups the measurement of the contractile properties on the musculus extensor digitorum longus muscle in the mdx mouse, in comparison to the untreated mdx mice (Example 1)

| | wild | |
|---|---|---|
| 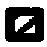 **A** | type | vehicle |
| | (BL10) | |
| 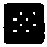 **B** | *mdx* | vehicle |
| 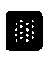 **C** | *mdx* | 1.0 mg/kg 5-amino-2,3-dihydro-1,4-phthalazinedione sodium salt |
| 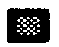 **D** | *mdx* | 2.5 mg/kg 5-amino-2,3-dihydro-1,4-phthalazinedione sodium salt |
| 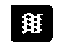 **E** | *mdx* | 5.0 mg/kg 5-amino-2,3-dihydro-1,4-phthalazinedione sodium salt |
| 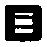 **F** | *mdx* | 1.0 mg/kg prednisolone |
| 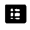 **G** | *mdx* | 1.0 mg/kg deflazacort |

Fig. 3: Bar diagrams of the body weight of the mice in the different treatment groups over the treatment period (weeks of age 5 - 10).

| | wild | |
|---|---|---|
| 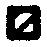 **A** | type | vehicle |
| | (BL10) | |
| 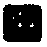 **B** | *mdx* | vehicle |
| 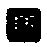 **C** | *mdx* | 1.0 mg/kg 5-amino-2,3-dihydro-1,4-phthalazinedione sodium salt |
| 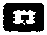 **D** | *mdx* | 2.5 mg/kg 5-amino-2,3-dihydro-1,4-phthalazinedione sodium salt |
| 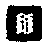 **E** | *mdx* | 5.0 mg/kg 5-amino-2,3-dihydro-1,4-phthalazinedione sodium salt |
| 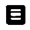 **F** | *mdx* | 1.0 mg/kg prednisolone |
| 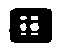 **G** | *mdx* | 1.0 mg/kg deflazacort |

## Claims

1. 5-amino-2,3-dihydro-1,4-phthalazinedione or one of its pharmaceutically acceptable salts for use in the treatment of a congenital muscular dystrophy, wherein said congenital muscular dystrophy is Duchenne muscular dystrophy or Becker muscular dystrophy.

2. 5-amino-2,3-dihydro-1,4-phthalazinedione or one of its pharmaceutically acceptable salts for use according to claim 1, wherein said pharmaceutically acceptable salt is 5-amino-2,3-dihydro-1,4-phthalazinedione sodium salt.

3. 5-amino-2,3-dihydro-1,4-phthalazinedione or one of its pharmaceutically acceptable salts for use according to claim 2, wherein 5-amino-2,3-dihydro-1,4-phthalazinedione sodium salt is provided as one of crystalline anhydrate polymorph forms I, II or III **characterized by** crystallography values determined by means of X-ray powder diagrams:
d values: 13.5; 6.9; 5.2; 4.6; 3.9; 3.5; 3.4; 3.3; 3.1; 3.0 and/or 2-theta values: 6.5; 12.7; 16.9; 19.3; 22.8; 25.8; 26.6; 27.2; 28.7; 30.3 for Form I,
d values: 12.9; 7.9; 7.1; 6.5; 5.3; 4.0; 3.7; 3.6; 3.3; 3.2 and/or 2-theta values: 6.8; 11.2; 12.5; 13.7; 16.7; 22.4; 24.3; 24.9; 27.2; 27.8 for Form II, and
d values: 13.131; 7.987; 7.186; 6.566; 6.512; 5.372; 3.994; 3.662; 3.406; 3.288; 3.283; 3.222; 3.215; 3.127; 2.889 and/or
2-theta values: 6.73; 11.07; 12.31; 13.48; 13.59; 16.49; 22.24; 24.29; 26.14; 27.10; 27.14; 27.67; 27.72; 28.52; 30.93 for Form Ill.

4. Pharmaceutical composition containing 5-amino-2,3-dihydro-1,4-phthalazinedione or one of its pharmaceutically acceptable salts as a single drug and at least one pharmaceutically acceptable excipient for use in the treatment of Duchenne muscular dystrophy or Becker muscular dystrophy.

5. Pharmaceutical composition for use according to claim 4, wherein said pharmaceutical composition is suitable for intravenous, oral, sublingual, inhalatory, rectal, topical or dermal administration.

6. Pharmaceutical composition for use according to any one of claims 4 or 5, wherein said at least one pharmaceutically acceptable excipient is selected from a group comprising carriers, binding agents, colorants, buffers, preservatives, antioxidants, coatings, sweeteners, thickening agents, pH-regulators, acidity regulators, acidifiers, solvents, isotonizing agents, disintegrants, glidants, lubricants, emulsifiers, solubilizing agents, stabilizers, diluents, anti-caking agents, sorbents, permeation enhancers, foaming agents, anti-foaming agents, opacifiers, fatliquors, consistency enhancers, hydrotropes, aromatic and flavoring substances.

7. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is applied orally in the form of tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

8. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is applied in the form of sublingual tablets or lozenges.

9. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is applied by inhalation by using a vibrant mesh nebulizer, metered dose-inhaler, jet nebulizer or dry-powder inhaler.

10. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is formulated as a retard drug.

11. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is formulated as a lyophilizate.

12. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is applied in form of liposomes, micelles, multilamellar vesicles or a cyclodextrin complex.

13. 5-amino-2,3-dihydro-1,4-phthalazinedione or one of its pharmaceutically acceptable salts for use according to claim 1 or a pharmaceutical composition for use according to claim 5, wherein a previous treatment with at least one other pharmaceutically active agent was refractory.

14. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is formulated as a suppository.

15. 5-amino-2,3-dihydro-1,4-phthalazinedione or one of its pharmaceutically acceptable salts for use according to claim 1 or a pharmaceutical composition for use according to claim 5, wherein said substance or said pharmaceutical composition is applied topically in the form of creams, emulsions, lotions, gels, hydrogels, pastes, powders, ointments, liniment, films, liposomes, dermal patches, transdermal patches, transdermal sprays or suspensions.

## Patentansprüche

1. 5-Amino-2,3-dihydro-1,4-phthalazindion oder eines seiner pharmazeutisch verträglichen Salze zur Verwendung bei der Behandlung einer angeborenen Muskeldystrophie, wobei es sich bei der angeborenen Muskeldystrophie um Duchenne-Muskeldystrophie oder Becker-Muskeldystrophie handelt.

2. 5-Amino-2,3-dihydro-1,4-phthalazindion oder eines seiner pharmazeutisch verträglichen Salze zur Verwendung gemäß Anspruch 1, wobei das besagte pharmazeutisch verträgliche Salz 5-Amino-2,3-dihydro-1,4-phthalazindion Natriumsalz ist.

3. 5-Amino-2,3-dihydro-1,4-phthalazindion oder eines seiner pharmazeutisch verträglichen Salze zur Verwendung gemäß Anspruch 2, wobei 5-Amino-2,3-dihydro-1,4-phthalazindion Natriumsalz als eine der kristallinen anhydrischen Polymorphe I, II oder III vorliegt, die durch mittels Röntgenpulverdiffraktogrammen ermittelte kristallographische Werte gekennzeichnet sind:
d-Werte: 13,5; 6,9; 5,2; 4,6; 3,9; 3,5; 3,4; 3,3; 3,1; 3,0 und/oder
2-Theta-Werte: 6,5; 12,7; 16,9; 19,3; 22,8; 25,8; 26,6; 27,2; 28,7; 30,3 für Form I,
d-Werte: 12,9; 7,9; 7,1; 6,5; 5,3; 4,0; 3,7; 3,6; 3,3; 3,2 und/oder
2-Theta-Werte: 6,8; 11,2; 12,5; 13,7; 16,7; 22,4; 24,3; 24,9; 27,2; 27,8 für Form II sowie
d-Werte: 13,131; 7,987; 7,186; 6,566; 6,512; 5,372; 3,994; 3,662; 3,406; 3,288; 3,283; 3,222; 3,215; 3,127; 2,889 und/oder
2-Theta-Werte: 6,73; 11,07; 12,31; 13,48; 13,59; 16,49; 22,24; 24,29; 26,14; 27,10; 27,14; 27,67; 27,72; 28,52; 30,93 für Form III.

4. Pharmazeutische Zusammensetzung, die 5-Amino-2,3-dihydro-1,4-phthalazindion oder eines seiner pharmazeutisch verträglichen Salze als einzigem Wirkstoff sowie mindestens einen pharmazeutisch verträglichen Hilfsstoff enthält, zur Verwendung bei der Behandlung von Duchenne-Muskeldystrophie oder Becker-Muskeldystrophie.

5. Pharmazeutische Zusammensetzung zur Verwendung gemäß Anspruch 4, wobei die pharmazeutische Zusammensetzung zur intravenösen, oralen, sublingualen, inhalativen, rektalen, topischen oder dermalen Verabreichung geeignet ist.

6. Pharmazeutische Zusammensetzung zur Verwendung gemäß einem der Ansprüche 4 oder 5, wobei der besagte mindestens eine pharmazeutisch verträgliche Hilfsstoff aus einer Gruppe ausgewählt ist, die Trägerstoffe, Bindemittel, Farbstoffe, Puffer, Konservierungsmittel, Antioxidantien, Beschichtungen, Süßungsmittel, Verdickungsmittel, pH-Regulatoren, Säureregulatoren, Säuerungsmittel, Lösungsmittel, Isotonisierungsmittel, Sprengmittel, Gleitmittel, Schmiermittel, Emulgatoren, Lösungsvermittler, Stabilisatoren, Verdünnungsmittel, Antibackmittel, Sorbenzien, Permeationsverstärker, Schaumbildner, Entschäumer, Trübungsmittel, Fettungsmittel, Konsistenzverstärker, Hydrotrope, Aromastoffe und Geschmacksstoffe umfasst.

7. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung oral in Form von Tabletten, Weichgelatinekapseln, Hartgelatinekapseln, zuckerbeschichteten Tabletten oder Pillen, Pulvern oder Granulaten, Säften, Sirups, Tropfen, Tees, Lösungen oder Suspensionen in wässrigen oder nichtwässrigen Flüssigkeiten, essbaren Schäumen oder Mousses, oder in Öl-in-Wasser- oder Wasser-in-Öl-Emulsionen verabreicht wird.

8. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung in Form von sublingualen Tabletten oder Lutschpastillen verabreicht wird.

9. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung durch Inhalation unter Verwendung eines Vibrating-Mesh-Verneblers, eines Dosierinhalators, eines Jet-Verneblers oder eines Trockenpulverinhalators verabreicht wird.

10. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung als Retardpräparat formuliert ist.

11. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung als Lyophilisat formuliert ist.

12. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung in Form von Liposomen, Mizellen, multilamellaren Vesikeln oder einem Cyclodextrin-Komplex verabreicht wird.

13. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei eine vorherige Behandlung mit mindestens einem anderen pharmazeutisch wirksamen Wirkstoff refraktär war.

14. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung als Suppositorium formuliert ist.

15. 5-Amino-2,3-dihydro-1,4-phthalazindion oder eines seiner pharmazeutisch verträglichen Salze zur Verwendung gemäß Anspruch 1 oder eine pharmazeutische Zusammensetzung zur Verwendung gemäß Anspruch 5, wobei die besagte Substanz oder die besagte pharmazeutische Zusammensetzung topisch in Form von Cremes, Emulsionen, Lotionen, Gelen, Hydrogelen, Pasten, Pulver, Salben, Einreibemitteln Filmen, Liposomen, Hautpflastern, transdermalen Pflastern, transdermalen Sprays oder Suspensionen angewendet wird.

## Revendications

1. 5-amino-2,3-dihydro-1,4-phtalazinedione ou l'un de ses sels pharmaceutiquement acceptables, destinée à être utilisée dans le traitement d'une dystrophie musculaire congénitale, ladite dystrophie musculaire congénitale étant la dystrophie musculaire de Duchenne ou la dystrophie musculaire de Becker.

2. 5-amino-2,3-dihydro-1,4-phtalazinedione ou l'un de ses sels pharmaceutiquement acceptables, destinée à être utilisée selon la revendication 1, ledit sel pharmaceutiquement acceptable étant le sel de sodium de 5-amino-2,3 -dihydro-1,4-phtalazinedione.

3. 5-amino-2,3-dihydro-1,4-phtalazinedione ou l'un de ses sels pharmaceutiquement acceptables, d'hydrates ou de solvates, destinés être utilisée selon la revendication 2, le sel de sodium de 5-amino-2,3-dihydro-1,4-phtalazinedione se présentant sous la forme de l'un des polymorphes cristallins anhydres I, Il ou III, **caractérisés par** des valeurs cristallographiques déterminées à l'aide de diffractogrammes de poudre aux rayons X :
valeurs d : 13,5 ; 6,9 ; 5,2 ; 4,6 ; 3,9 ; 3,5 ; 3,4 ; 3,3 ; 3,1 ; 3,0 et/ou
valeurs 2-thêta : 6,5 ; 12,7 ; 16,9 ; 19,3 ; 22,8 ; 25,8 ; 26,6 ; 27,2 ; 28,7 ; 30,3 pour la forme I,
valeurs d : 12,9 ; 7,9 ; 7,1 ; 6,5 ; 5,3 ; 4,0 ; 3,7 ; 3,6 ; 3,3 ; 3,2 et/ou
valeurs 2-thêta : 6,8 ; 11,2 ; 12,5 ; 13,7 ; 16,7 ; 22,4 ; 24,3 ; 24,9 ; 27,2 ; 27,8 pour la forme Il ainsi que
valeurs d : 13,131 ; 7,987 ; 7,186 ; 6,566 ; 6,512 ; 5,372 ; 3,994 ; 3,662 ; 3,406 ; 3,288 ; 3,283 ; 3,222 ; 3,215 ; 3,127 ; 2,889 et/ou
valeurs 2-thêta : 6,73 ; 11,07 ; 12,31 ; 13,48 ; 13,59 ; 16,49 ; 22,24 ; 24,29 ; 26,14 ; 27,10 ; 27,14 ; 27,67 ; 27,72 ; 28,52 ; 30,93 pour la forme III.

4. Composition pharmaceutique contenant de 5-amino-2,3-dihydro-1,4-phtalazinedione ou l'un de ses sels pharmaceutiquement acceptables comme seul principe actif, ainsi qu'au moins un excipient pharmaceutiquement acceptable, destinée à être utilisée dans le traitement de la dystrophie musculaire de Duchenne ou de la dystrophie musculaire de Becker.

5. Composition pharmaceutique destinée à être utilisée selon la revendication 4, ladite composition pharmaceutique étant adaptée à une administration intraveineuse, orale, sublinguale, par inhalation, rectale, topique ou cutanée.

6. Composition pharmaceutique destinée à être utilisée selon l'une des revendications 4 ou 5, dans laquelle ledit au moins un excipient pharmaceutiquement acceptable est choisi parmi un groupe comprenant véhicules, liants, colorants, tampons, conservateurs, antioxydants, revêtements, édulcorants, épaississants, régulateurs de pH, régulateurs d'acidité, acidifiants, solvants, agents isotonisants, agents désintégrants, agents glissants, lubrifiants, émulsifiants, agents solubilisants, stabilisants, diluants, agents anti-agglomérants, sorbants, promoteurs de perméation, agents moussants, agents antimoussants, agents opacifiants, agents de retannage, promoteurs de consistance, hydrotropes ainsi que des arômes et des substances aromatiques.

7. 5-amino-2,3-dihydro-1,4-phtalazinedione ou l'un de ses sels pharmaceutiquement acceptables, destinée à être utilisée selon la revendication 1, ou une composition pharmaceutique destinée à être utilisée selon la revendication 5, ladite substance ou ladite composition pharmaceutique pouvant être administrée par voie orale sous forme de comprimés, de gélules en gélatine molle, de gélules en gélatine dure, de comprimés ou pilules revêtis de sucre, de poudres ou de granulés, de jus, de sirops, de gouttes, de tisanes, de solutions ou de suspensions dans des milieux aqueux ou non aqueux, de préparations moussantes, de mousses comestibles, ou dans des émulsions huile-dans-eau ou eau-dans-huile.

8. 5-amino-2,3-dihydro-1,4-phtalazinedione ou l'un de ses sels pharmaceutiquement acceptables, destinée à être utilisée selon la revendication 1, ou une composition pharmaceutique destinée à être utilisée selon la revendication 5, ladite substance ou ladite composition pharmaceutique étant administrée sous forme de comprimés sublinguaux ou de pastilles à sucer.

9. 5-amino-2,3-dihydro-1,4-phtalazinedione ou l'un de ses sels pharmaceutiquement acceptables, destinée à être utilisée selon la revendication 1, ou une composition pharmaceutique destinée à être utilisée selon la revendication 5, ladite substance ou ladite composition pharmaceutique étant administrée par inhalation à l'aide d'un nébuliseur à maille vibrante, d'un inhalateur-doseur, d'un nébuliseur à jet ou d'un inhalateur à poudre sèche.

10. 5-amino-2,3-dihydro-1,4-phtalazinedione ou l'un de ses sels pharmaceutiquement acceptables, destinée à être utilisée selon la revendication 1, ou une composition pharmaceutique destinée à être utilisée selon la revendication 5, ladite substance ou ladite composition pharmaceutique étant formulée sous forme de préparation à action retardée.

11. 5-amino-2,3-dihydro-1,4-phtalazinedione ou l'un de ses sels pharmaceutiquement acceptables, destinée à être utilisée selon la revendication 1, ou une composition pharmaceutique destinée à être utilisée selon la revendication 5, ladite substance ou ladite composition pharmaceutique étant formulée sous forme de lyophilisat.

12. 5-amino-2,3-dihydro-1,4-phtalazinedione ou l'un de ses sels, hydrates ou solvates pharmaceutiquement acceptables, destinée à être utilisée selon la revendication 1, ou une composition pharmaceutique destinée à être utilisée selon la revendication 5, ladite substance ou ladite composition pharmaceutique étant administrée sous forme de liposomes, de micelles, de vésicules multilamellaires ou d'un complexe de cyclodextrine.

13. 5-amino-2,3-dihydro-1,4-phtalazinedione ou l'un de ses sels, hydrates ou solvates pharmaceutiquement acceptables, destinée à être utilisée selon la revendication 1, ou une composition pharmaceutique destinée à être utilisée selon la revendication 5, un traitement préalable par au moins un autre principe actif pharmaceutique ayant été réfractaire.

14. 5-amino-2,3-dihydro-1,4-phtalazinedione ou l'un de ses sels pharmaceutiquement acceptables, destinée à être utilisée selon la revendication 1, ou une composition pharmaceutique destinée à être utilisée selon la revendication 5, ladite substance ou ladite composition pharmaceutique étant formulée sous forme de suppositoire.

15. 5-amino-2,3-dihydro-1,4-phtalazinedione ou l'un de ses sels, hydrates ou solvates pharmaceutiquement acceptables, destinée à être utilisée selon la revendication 1, ou une composition pharmaceutique destinée à être utilisée selon la revendication 5, ladite substance ou ladite composition pharmaceutique étant appliquée par voie topique sous forme de crèmes, d'émulsions, de lotions, de gels, d'hydrogels, de pâtes, de poudres, de pommades, de liniments, de films, de liposomes, de patchs cutanés, de patchs transdermiques, de sprays transdermiques ou de suspensions.
